(19) Europäisches Patentamt
European Patent Office
Office européen des brevets

(11) Veröffentlichungsnummer: **0 377 083 B1**

# (12) EUROPÄISCHE PATENTSCHRIFT

(45) Veröffentlichungstag der Patentschrift: **22.09.93**

(51) Int. Cl.5: **C12P 13/04**, //C12N9/86

(21) Anmeldenummer: **89118193.5**

(22) Anmeldetag: **30.09.89**

## (54) Verfahren zur Herstellung von L-alpha-Aminosäuren.

(30) Priorität: **02.01.89 DE 3900007**

(43) Veröffentlichungstag der Anmeldung:
**11.07.90 Patentblatt 90/28**

(45) Bekanntmachung des Hinweises auf die Patenterteilung:
**22.09.93 Patentblatt 93/38**

(84) Benannte Vertragsstaaten:
**AT BE CH DE ES FR GB GR IT LI LU NL SE**

(56) Entgegenhaltungen:
**EP-A- 0 159 866**
**EP-A- 0 309 310**
**DE-A- 2 811 303**
**FR-A- 2 393 848**

(73) Patentinhaber: **RÜTGERSWERKE AKTIENGESELLSCHAFT**
**Mainzer Landstrasse 217**
**D-60326 Frankfurt(DE)**

(72) Erfinder: **Wagner, Fritz, Prof. Dr.**
**Hohe Wiese 2**
**D-3300 Braunschweig(DE)**
Erfinder: **Syldatk, Christoph, Dr.**
**Ernst-Heilmann-Grund 2**
**D-3200 Hildesheim(DE)**
Erfinder: **Lehmensiek, Vera**
**Buchenberg 8b**
**D-3305 Dettum(DE)**
Erfinder: **Krohn, Karsten, Prof. Dr.**
**Am Turmsberg 49**
**D-3300 Braunschweig(DE)**
Erfinder: **Höke, Hartmut, Dr.**
**Erfurter Strasse 89**
**D-4620 Castrop Rauxel(DE)**
Erfinder: **Läufer, Albrecht, Dr.**
**Maybachstrasse 5**
**D-4300 Essen(DE)**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



Rank Xerox (UK) Business Services
(3.10/3.6/3.3.1)

## Beschreibung

Die Erfindung betrifft ein neues, mikrobiell-enzymatisches Verfahren zur Herstellung von L-alpha-Aminosäuren aus 5-substituierten Hydantoinen.

L-alpha-Aminosäuren werden als Zusatz für Futter- und Nahrungsmittel sowie zur Herstellung pharmazeutischer Produkte und als Zwischenprodukt für chemische Synthesen verwendet.

Nach DE 37 12 539 C2 können L-alpha-Aminosäuren durch biochemische Umsetzung mit Hilfe von Mikroorganismen aus 5-substituierten Hydantoinen oder N-Carbamoyl-alpha-Aminosäuren gewonnen werden. Optimierungsversuche mit den in dieser Patentschrift beschriebenen Stämmen haben gezeigt, daß das Wachstum und die Enzymsynthese durch Zusatz von Glucose und Ammoniumsulfat und Hefeextrakt zum Medium günstig beeinflußt wird. Die Ausprägung einer hohen spezifischen Enzymaktivität ist allerdings an die Anwesenheit eines geeigneten Induktors und von Manganionen im Medium gebunden, da erst unter Einfluß eines entsprechenden Induktors die Organismen beginnen, die an der Umsetzung beteiligten Enzyme wie Hydantoin-Racemase, Hydantoinase und L-N-Carbamoylase zu bilden. Gemäß DE 37 12 539 C2 kann D,L-3'-Methylenindolyl-5-hydantoin sowohl als Induktor als auch als Kohlenstoffquelle dienen.

Daher reicht es jedoch in einem Batch-Ansatz nicht aus, diesen Induktor zu Beginn dem Medium zuzufügen, da seine Wirkung mit der Zeit abnimmt. Erst erneute Zudosierung führt wieder zu einem Anstieg der Aktivität. Auch die Induktorzudosage in der späten Wachstumsphase über 4 - 5 Stunden erwies sich als erfolgreich; trotzdem bedeutet dies einen unerwünscht hohen Induktorverbrauch. Außerdem entstehen Produkte, die sich auf das Wachstum hemmend auswirken und das Medium sichtbar verfärben. Zur Isolierung der gebildeten L-alpha-Aminosäure müssen diese Abbauprodukte zusätzlich abgetrennt werden, was einen erhöhten Reinigungsaufwand bedingt.

Es ist ein weiterer Nachteil, daß bei diesem Induktor dem Kulturmedium ein organisches Lösungsmittel wie z. B. Polyethylenglykol eingesetzt werden muß.

Die Aufgabe der vorliegenden Erfindung besteht daher darin, das bekannte Verfahren zur Herstellung von L-alpha-Aminosäuren durch Bereitstellung eines Induktors zu verbessern, der während der mikrobiellen Umsetzung möglichst nicht abgebaut wird, der keine das Wachstum und die Enzymaktivität hemmende Produkte bildet, der bei der Aufarbeitung des Reaktionsgemisches keinen zusätzlichen Reinigungsaufwand erfordert, und der auch ohne zusätzliches Lösungsmittel eine hohe Ausbeute an L-alpha Aminosäuren ermöglicht.

Die Lösung der Aufgabe erfolgt durch ein Verfahren gemäß der Ansprüche 1-4.

Es wurde gefunden, daß Verbindungen mit der Struktur

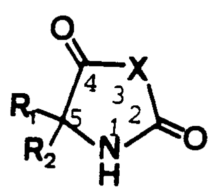

wobei $R_1$ und $R_2$ gleich oder verschieden sind,

$R_1$ = H oder eine Alkylgruppe mit 1-4 C-Atomen,

$R_2$ = eine Alkylgruppe mit 1-4 C-Atomen oder die folgenden Reste

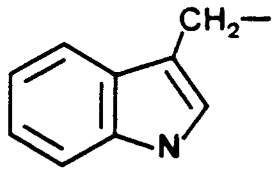

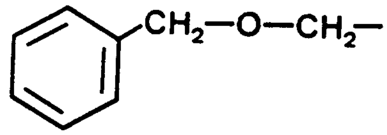

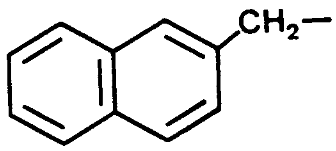

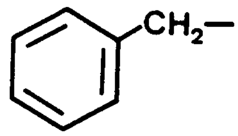

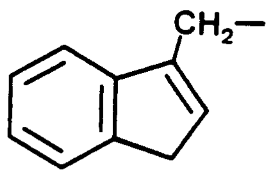

und X = **>N-Alk** oder **>CH-Alk** oder **>$CH_2$** ,

wobei Alk eine verzweigte oder unverzweigte Alkylkette mit 1-4 C-Atomen darstellt, als hochwirksame Induktoren eingesetzt werden können.

Überraschenderweise werden diese trotz ihrer guten Induktionswirkung während der Kultivierung nicht merklich abgebaut. Eine Wachstumshemmung durch Anreicherung von Abbauprodukten tritt nicht ein, und somit ist der Bedarf an Induktor wesentlich reduziert. Weiterhin wird durch den erfindungsgemäßen Induktor eine Verfahrensvereinfachung erzielt, da der Induktor einmalig zu Beginn oder im Verlauf der Biomasseanzucht vorgelegt wird und seine Aktivität bis zum Ende erhalten bleibt. Trotzdem kann gleichzeitig eine höhere Enzymaktivität festgestellt werden.

In Tabelle 1 wird der Einfluß der Induktoren von D,L-3'-Methylenindolyl-5-hydantoin und 3-N-Methyl-D,L-3'-methylenindolyl-5-hydantoin miteinander verglichen.

Die Anzucht erfolgte im Bioreaktor im Mineralsalzmedium mit Glucosevorlage und -zudosierung über 16 Stunden gemäß Beispiel 1.

Die Enzymaktivitäten wurden nach verschiedenen Kultivierungszeiten gemäß Beispiel 2 gemessen. Im Vergleich zu D,L-3'-methylenindolyl-5-hydantoininduzierten Zellen wiesen 3-N-Methyl-D,L-3'-methylenindolyl-5-hydantoininduzierte Zellen eine etwa 5fach höhere spezifische Aktivität auf.

Tabelle 1: Wachstum und Enzyminduktion bei Coryneforme-Bakterien DSM 3747 nach 16 h im 20 l-Bioreaktor

| vorgelegter Induktor: | | 1.0 g/l D,L-5-IMH | 1.0 g/l 3-N-$CH_3$-D,L-5-IMH |
|---|---|---|---|
| X (g/l) | | 14 | 15 |
| $Y_{x/s}$ | | 0.35 | 0.40 |
| $\mu$ ($h^{-1}$) | | 0.22 | 0.27 |
| Spezifische | Trp | 0.047 | 0.230 |
| Aktivität | CTrp | 0.035 | 0.037 |
| (mmol/gBTMxh) | Trp+CTrp | 0.082 | 0.267 |

Trp = L-Tryptophan
CTrp = N-Carbamoyl-D,L-tryptophan
X = Biotrockenmasse (g/l)

$$Y_{x/s} = \text{Ertragskoeffizient} = \frac{X\ (g/l)}{\text{Substrat}\ (g/l)}$$

$\mu$ = spezifische Wachstumsrate (1/h)
D,L-5-IMH = D,L-3'-Methylenindolyl-5-hydantoin
3-N-$CH_3$-D,L-5-IMH = 3-N-Methyl-D,L-3'-methylenindolyl-5-hydantoin

Bei einer einmaligen Vorlage von 1,0 g/l 3-N-Methyl-D,L-3'-methylenindolyl-5-hydantoin im Nährmedium kann bei einer Biotrockenmasse (BTM) von 29 g/l mit einer spezifischen Aktivität von 0,352 (mmol/g BTMxh) Trp, 0,039 (mmol/g BTMxh) CTrp und 0,391 (mmol/g BTMxh) Trp + CTrp in 29 h erhalten werden.

Die Konzentration dieses neuen Induktors bleibt während des gesamten Zeitraums konstant. Mit D,L-3'-Methylenindolyl-5-hydantoin als Induktor können dagegen spezifische Aktivitäten von nur 0,107 (mmol/g BTMxh) Trp, 0,025 (mmol/g BTMxh) CTrp und 0,132 (mmol/g BTMxh) Trp + CTrp erreicht werden.

Bei 3-N-Methyl-D,L-3'-methylenindolyl-5-hydantoin als Induktor wird eine 3- bis 5fach höhere Enzymaktivität erzielt und das Produktverhältnis wird deutlich zugunsten der Tryptophanbildung verschoben. Erfindungsgemäß einsetzbare Induktoren sind Verbindungen der allgemeinen Struktur

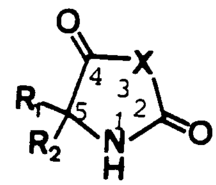

wobei $R_1$ und $R_2$ gleich oder verschieden sind,

$R_1$ = H oder eine Alkylgruppe mit 1-4 C-Atomen,

$R_2$ = eine Alkylgruppe mit 1-4 C-Atomen oder die folgenden Reste

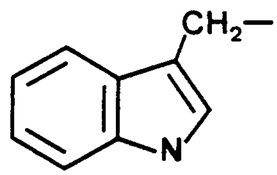

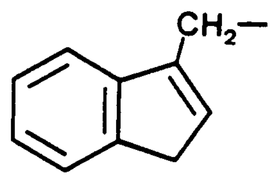

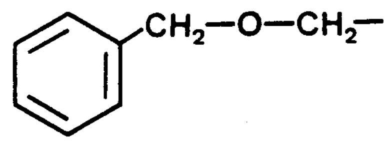

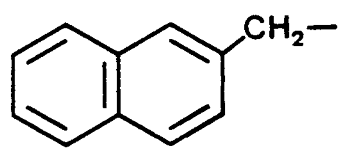

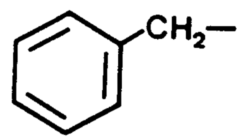

und X = **>N-Alk** oder **>CH-Alk** oder **>$CH_2$** ,

wobei Alk eine verzweigte oder unverzweigte Alkylkette mit 1-4 C-Atomen darstellt.

Es sind demnach Hydantoine oder Diketopyrrolidine, die sowohl in 3- als auch in 5-Stellung substituiert sind. Mögliche Substituenten in 3-Stellung sind niedrige Alkylgruppen wie z. B. die Methyl-, Ethyl-, n-Propyl-, i-Propyl-, n-Butyl-, i-Butyl- und tert. Butylgruppe. Die Position 5 kann mono- oder disubstituiert sein, wobei die Substituenten $R_1$ und $R_2$ gleiche niedrige Alkylgruppen wie die Methyl-, Ethyl-, n-Propyl-, i-Propyl-, n-Butyl-, i-Butyl- und tert. Butylgruppe sind oder $R_1$ Wasserstoff oder eine niedrige Alkylgruppe und $R_2$ eine niedrige Alkylgruppe oder eine Gruppe der folgenden Struktur ist:

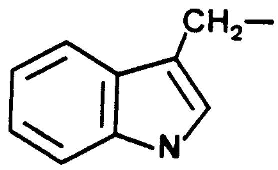

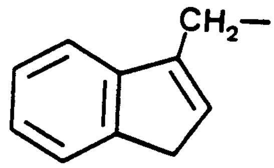

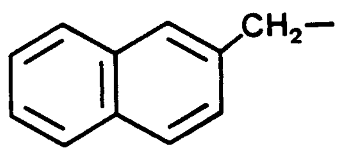

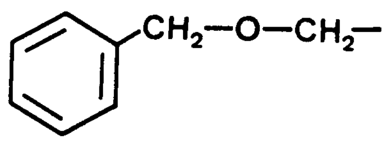

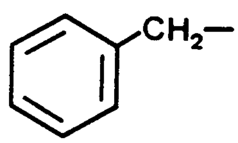

Bevorzugte Induktoren sind in 3-Stellung alkylierte Hydantoine mit einem Aralkylrest in Position 5. Diese Induktoren werden dem Kulturmedium bereits zu Beginn oder im Laufe der Anzucht der Mikroorganismen einmalig in einer Menge von 0,1 bis 2,0 g/l zugegeben.

Die Anzucht der Mikroorganismen und die biochemische Hydantoinspaltung erfolgen in an sich bekannter Weise, wie sie z. B. in DE 37 12 539 C2 beschrieben sind.

Im erfindungsgemäßen Verfahren können alle Mikroorganismen eingesetzt werden, die substituierte Hydantoine in der gewünschten Weise, bevorzugt solche die Hydantoine enantioselektiv zu der entsprechenden L-Form spalten. Bekannt ist diese Spaltung auch noch bei Mikroorganismen der Gattungen Arthrobacter, Flavobakterium und Pseudomonas.

Dem erfindungsgemäßen Verfahren zugänglich sind alle 5-substituierten Hydantoine, die durch an sich bekannte Umsetzungen zugänglich sind.

Beispiele für 5-substituierte Hydantoine, die mit den erfindungsgemäßen Mikroorganismen über N-Carbamoyl-alpha-Aminosäuren zu L-alpha-Aminosäuren hydrolysiert werden können, sind in Tabelle 2 aufgeführt. Die Erfindung ist jedoch nicht auf diese Beispiele beschränkt.

Tabelle 2

| 5-substituierte Hydantoine und Abbauprodukte | | | | |
|---|---|---|---|---|
| 5-Substituent | N-Carbamoylaminosäure | | alpha-Aminosäure | |
| | % D*) | % L*) | % D*) | % L*) |
| Ethylen-2'-methylthioether | 100 | 0 | 0 | 100 |
| Benzyl | 50 | 50 | 0 | 100 |
| p-Hydroxybenzyl | 56 | 44 | 0 | 100 |
| 3,4-Dihydroxybenzyl | 58 | 42 | 0 | 100 |
| Benzylmethylenether | 50 | 50 | 0 | 100 |
| 3'-Methylenindol | 6 | 94 | 0 | 100 |

*) Konfiguration

Beispiel für N-Carbamoyl-alpha-Aminosäuren, die mit den erfindungsgemäß eingesetzten Mikroorganismen zu L-alpha-Aminosäuren gespalten werden können, sind in Tabelle 3 aufgeführt.

Tabelle 3

| Substrat | Produkt |
|---|---|
| N-Carbamoyl-D,L-tryptophan | L-Tryptophan |
| N-Carbamoyl-L-tryptophan | L-Tryptophan |
| N-Carbamoyl-D,L-phenylalanin | L-Phenylalanin |
| N-Carbamoyl-D-phenylalanin | L-Phenylalanin |
| N-Carbamoyl-D-methionin | L-Methionin |

## Beispiele

Beispiel 1

Kultivierungsbedingungen

Gemäß DE 37 12 539 C2 wird eine Vorkultur der Organismen des hinterlegten Stammes (DSM 3747) hergestellt. Für die Hauptkultur sind folgende Bedingungen gegeben:

| | | |
|---|---|---|
| Glucosevorlage: | 20 | g/l |
| Glucosezudosage: | 3-5 | g/l $h^{-1}$ ab 10. Stunde |
| techn. Hefeextrakt: | 1,0 | g/l |
| $KH_2PO_4$: | 3,4 | g/l |
| $Na_2HPO_4 \times 2H_2O$: | 4,41 | g/l |
| Zitronensäure x $1H_2O$: | 0,64 | g/l |
| $MgSO_4 \times 7\ H_2O$: | 0,40 | g/l |
| $CaCl_2 \times 2H_2O$: | 0,04 | g/l |
| $FeSO_4 \times 7\ H_2O$: | 0,04 | g/l |
| $MnCl_2 \times 4H_2O$: | 0,04 | g/l |
| $(NH_4)_2SO_4$: | 6,5 | g/l |
| Induktor*: | 1,0 | g/l |
| pH-Wert: | 7,0 | über $NH_3$-Titration (10 % wässrige Lösung) |
| Temperatur: | 30 °C | |
| Kultivierungsdauer: | 15 - 30 h | |

| | | |
|---|---|---|
| * a) D,L-5-IMH: | 1,0 | g/l(Vorlage) + Zudosage (in Abhängigkeit von der Kultivierungsdauer) |
| b) 3-N-$CH_3$-D,L-5-IMH: | 1,0 | g/l (neuer Induktor) |

Beispiel 2

Enzymaktivitätstest

Proben werden zu definierten Kultivierungszeiten entnommen und zentrifugiert.

100 mg Biofeuchtmasse wird in abgeschlossenen 10 ml-Schüttelkolben in 5 ml 0,1 M Glycin-Puffer oder 0,05 M Phosphatpuffer (pH 8,5) suspendiert und mit 5 mg D,L-5-IMH bei 27 °C 2 h lang unter $N_2$-Atmosphäre geschüttelt.

Die Konzentration an IMH, N-Carbamoyltryptophan und L-Tryptophan wird aus dem Überstand per HPLC (RP-8-Säule, Serva, Heidelberg; 50 mM $KH_2$ $PO_4$/25 % Methanol als Fließmittel sowie UV-Detektion bei 280 nm; Flußrate 1,0 ml/min) bestimmt.

Die Retentionszeiten sind:

| | |
|---|---|
| Trp: | 6,1 min |
| CTrp: | 8,5 min |
| IMH: | 16,7 min |

Beispiel 3

Der Biokatalysator wird gemäß Beispiel 1 mit den jeweiligen Induktoren angezogen und nach 20 h abzentrifugiert.

120 g Biofeuchtmasse werden in 2 l Carbonatpuffer (0,1 m, pH 8,5 geregelt) und 20,0 g (87,2 mmolar) D,L-5-IMH als Substrat bei 50 °C gerührt nach einmaliger $N_2$-Begasung.

a) D,L-5-IMH-induzierte Zellen setzen D,L-5-IMH nach 3 h vollständig um. Zwischenzeitlich entsteht über 2 h deutlich CTrp als Reaktionsprodukt, d. h. es erfolgt keine vollständige Umsetzung, da im 2. Reaktionsschritt die L-N-Carbamoylase limitierend wirkt (Tabelle 4).

b) $3\text{-}N\text{-}CH_3$-D,L-5-IMH-induzierte Zellen setzen D,L-5-IMH innerhalb 1 h um. Der zweite Reaktionsschritt L-N-Carbamoylase verläuft deutlich schneller (Tabelle 4).

Tabelle 4

Konzentrationen an Zwischenprodukt (CTrp) und Produkt (L-Trp) sowie Ausbeute (% L-Trp) nach Induktion mit D,L-5-IMH bzw. $3\text{-}N\text{-}CH_3$-D,L-5-IMH und Umsetzung mit D,L-5-IMH als Substrat im Kleinreaktor:

| Reaktionszeit (h) | D,L-5-IMH | | | $3\text{-}N\text{-}CH_3$-D,L-5-IMH | | |
|---|---|---|---|---|---|---|
| | CTrp (mM) | Trp (mM) | % Trp | CTrp (mM) | Trp (mM) | % Trp |
| 0 | 0 | 0 | 0 | 0 | 0 | 0 |
| 1,0 | 14,1 | 56,3 | 64 | <1 | >86 | >99 |
| 1,5 | 11,1 | 74,4 | 85 | <1 | >86 | >99 |
| 2,0 | 8,0 | 81,4 | 93 | | | |
| 3,0 | 3,2 | 82,4 | 94 | | | |

## Patentansprüche

1. Verfahren zur Herstellung von L-alpha-Aminosäuren, durch mikrobielle Spaltung von 5-substituierten Hydantoinen, **dadurch gekennzeichnet**, daß als Induktor für die Enzymaktivität Verbindungen der Struktur

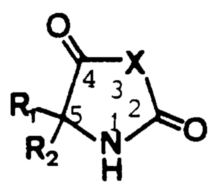

wobei $R_1$ und $R_2$ gleich oder verschieden sind,

$R_1$ = H oder eine Alkylgruppe mit 1-4 C-Atomen,

$R_2$ = eine Alkylgruppe mit 1-4 C-Atomen oder die folgenden Reste

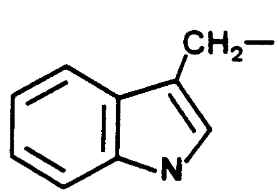
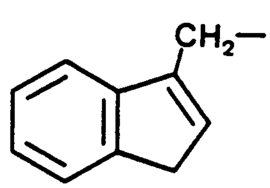
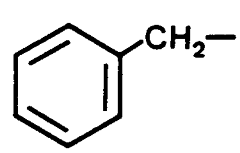
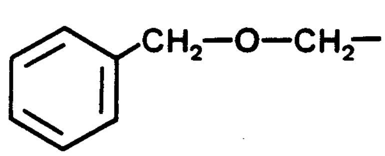
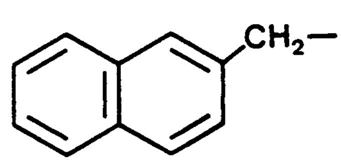

und X = **>N-Alk** oder **>CH-Alk** oder **>$CH_2$** ,
wobei Alk eine verzweigte oder unverzweigte Alkylkette mit 1-4 C-Atomen darstellt, eingesetzt werden.

**2.** Verfahren nach Anspruch 1, **dadurch gekennzeichnet**, daß als Induktor Verbindungen der Struktur

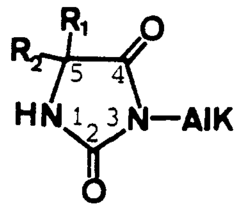

wobei $R_1$, $R_2$ und Alk die in Anspruch 1 genannte Bedeutung haben, eingesetzt werden.

**3.** Verfahren nach den Ansprüchen 1 und 2, **dadurch gekennzeichnet**, daß als Induktor 3-N-Methyl-D,L-3'-methylenindolyl-5-hydantoin eingesetzt wird.

**4.** Verfahren nach den Ansprüchen 1-3, **dadurch gekennzeichnet**, daß der Induktor nur einmal bei der Anzucht der Mikroorganismen zugegeben wird.

**Claims**

**1.** A process for preparing L-alpha-amino acids by microbial splitting of 5-substituted hydantoins, **characterized in that** the inducer used for the enzyme activity is in the form of compounds of the structure:

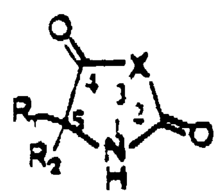

in which **$R_1$** and **$R_2$** are the same or different,
**$R_1$** = H or an alkyl group with 1-4 C-atoms,
**$R_2$** = an alkyl group with 1-4 C-atoms or the following residues:

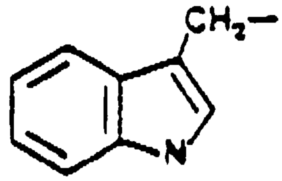
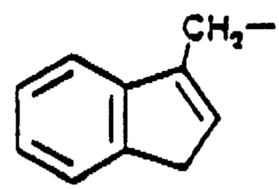
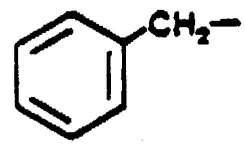
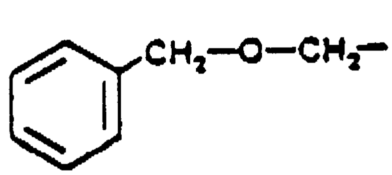
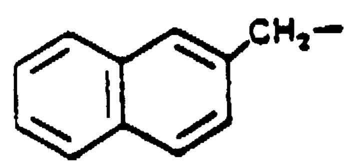

and **X** = **>N-Alk** or **>CH-Alk** or **>$CH_2$**,
in which **Alk** represents a branched or unbranched alkyl chain with 1-4 C-atoms.

2. A process according to Claim 1, **characterized in that** the inducer used is in the form of compounds of the structure:

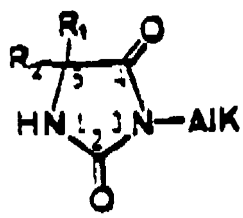

in which $\mathbf{R_1}$, $\mathbf{R_2}$ and **Alk** have the values indicated in Claim 1.

3. A process according to Claims 1 and 2, **characterized in that** 3-N-methyl-D,L-3'-methyleneindolyl-5-hydantoin is used as the inducer.

4. A process according to Claims 1 to 3, **characterized in that** the inducer is added only once during the cultivation of the micro-organisms.

**Revendications**

1. Procédé de préparation de L-alpha-amino-acides par scission microbienne d'hydantoïnes substituées en position 5, caractérisé en ce qu'on met en oeuvre comme inducteur pour l'activité enzymatique des composés de structure

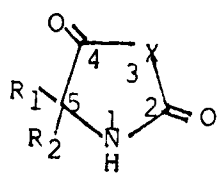

où $R_1$ et $R_2$ sont identiques ou différents, $R_1$ = H ou un groupe alkyle avec 1 à 4 atomes de C, $R_2$ = un groupe alkyle avec 1 à 4 atomes de C ou les restes suivants

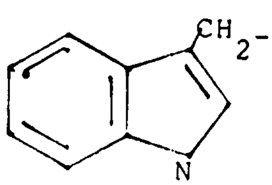

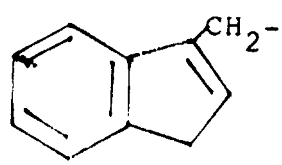

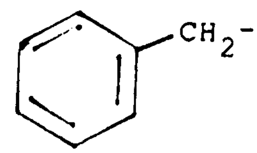

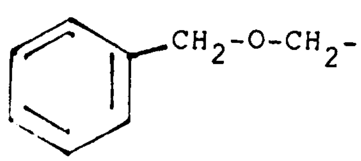

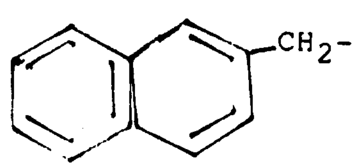

et X = >N-Alk ou >CH-Alk ou >$CH_2$, Alk représentant une chaîne alkyle ramifiée ou non ramifiée avec 1 à 4 atomes de C.

2. Procédé selon la revendication 1, caractérisé en ce qu'on met en oeuvre comme inducteur des composés de structure

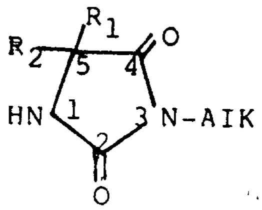

où $R_1$, $R_2$ et Alk ont la signification indiquée dans la revendication 1.

**3.** Procédé selon les revendications 1 et 2, caractérisé en ce qu'on met en oeuvre comme inducteur de la 3-N-méthyl-D,L-3'-méthylèneindolyl-5-hydantoïne.

**4.** Procédé selon les revendications 1 à 3, caractérisé en ce que l'inducteur n'est ajouté qu'une seule fois lors de la culture des micro-organismes.